# EUROPÄISCHE PATENTSCHRIFT

(11) **EP 0 971 912 B1**
(45) Veröffentlichungstag und Bekanntmachung des Hinweises auf die Patenterteilung: **14.08.2002**
(21) Anmeldenummer: 98905372.3
(22) Anmeldetag: 30.01.1998
(51) Int. Cl.: C07D 307/89

(54) **VERFAHREN ZUM BEHANDELN VON ROHEM PHTHALSÄURE-ANHYDRID**
PROCESS FOR TREATING CRUDE PHTHALIC ANHYDRIDE
PROCEDE DE TRAITEMENT D'ANHYDRIDE PHTALIQUE BRUT

(30) Priorität: 13.03.1997 DE 19710429
(43) Veröffentlichungstag der Anmeldung: 19.01.2000
(73) Patentinhaber: MG Technologies AG, 60325 Frankfurt am Main (DE)
(72) Erfinder: FRANZ, Volker, D-60486 Frankfurt am Main (DE); SIEBERT, Wolfgang, D-60322 Frankfurt am Main (DE); DOMES, Helmuth, D-63179 Obertshausen (DE)
(74) Vertreter: Revesz, Veronika
(86) Internationale Anmeldenummer: EP9800495
(87) Internationale Veröffentlichungsnummer: WO9840372

(56) Entgegenhaltungen:
- GB-A- 787 924
- GB-A- 1 126 540
- US-A- 2 076 033
- US-A- 4 435 580

## Beschreibung

Die Erfindung betrifft ein Verfahren zum Behandeln von rohem Phthalsäure-Anhydrid (PSA), welches katalytisch aus sauerstoffhaltigem Gas und Orthoxylol oder Naphthalin bei Temperaturen im Bereich von 300 bis 500 ° C in einem Reaktor erzeugt und in einem Gase und Dämpfe enthaltenden Gemisch aus dem Reaktor abgezogen wird, wobei man das Gemisch kühlt und flüssiges PSA durch eine thermische Vorbehandlung und eine destillative Reinigung leitet.

Ein Verfahren dieser Art ist aus der DE-C-3 538 911 bekannt. Hierbei wird das rohes PSA enthaltende dampfförmige Gemisch an mindestens einem von mehreren Festabscheidem gekühlt, festes PSA wieder verflüssigt und vor der destillativen Reinigung durch einen oder mehrere Behälter geführt, in denen die thermische Vorbehandlung stattfindet. In der US-A-4 592 412 ist die Arbeitsweise von Festabscheidern beschrieben.

Die GB-A-1 126 540 offenbart ein Verfahren zur Reinigung von PSA, wobei das PSA-haltige Gemisch in Form von Gasen und Dämpfen durch einen Flüssigabscheider in einen flüssigen Teil und ein restliches Gemisch aus Gasen getrennt wird. Das restliche Gemisch aus Gasen wird in einem Festabscheider gekühlt um das PSA in fester Form zu erhalten.

Der Erfindung liegt die Aufgabe zugrunde, die Ausbeute und die Qualität des erzeugten PSA-Produkts zu verbessern und dabei kostengünstig zu arbeiten. Erfindungsgemäß gelingt dies beim eingangs genannten Verfahren dadurch, dass man
a) das Gase und Dämpfe enthaltende, aus dem Reaktor abgezogene PSA-haltige Gemisch zunächst durch einen gekühlten, kontinuierlich arbeitenden Flüssigabscheider leitet und einen ersten flüssigen, PSA-reichen Strom und, getrennt davon, ein restliches Gemisch aus Gasen und Dämpfen abzieht,
b) das restliche, PSA-haltige Gemisch durch mindestens einen von mehreren gekühlten Festabscheidern führt, wobei PSA in fester Form abgeschieden und wieder verflüssigt als zweiter PSA-reicher Strom aus den Festabscheidern abgezogen wird und wobei das Verhältnis der Mengen pro Zeiteinheit des ersten und zweiten Stroms 1 : 5 bis 2 : 1 beträgt,
c) den ersten PSA-reichen Strom bei Temperaturen von 250 bis 300 ° C zur thermischen Vorbehandlung unter Rühren durch einen ersten Verweilzeitbehälter leitet, wobei die Verweilzeit im ersten Behälter mindestens drei Stunden beträgt, und
d) den zweiten PSA-reichen Strom unter Umgehung des ersten Verweilzeitbehälters zusammen mit dem thermisch vorbehandelten ersten Strom zur destillativen Reinigung führt. Die Menge des zweiten Stroms ist die Summe der aus den Festabscheidern kommenden Teilströme.

Beim Verfahren der Erfindung gibt man dem ersten Verweilbehälter nur den aus dem Flüssigabscheider kommenden ersten Strom auf. Im Flüssigabscheider wird das aus dem Reaktor kommende Produktgemisch nur soweit gekühlt, daß flüssiges PSA entsteht, wobei man feste Anbackungen vermeidet. Der an sich bekannte Flüssigabscheider arbeitet z. B. wie ein Rippenrohr-Wärmeaustauscher. Demgegenüber werden in den Festabscheidern durch Kühlen anhaftende PSA-Beläge gebildet, die dann periodisch wieder abgeschmolzen werden. Dabei arbeiten die Festabscheider im zeitversetzten Zyklus zueinander, einmal in der Abscheidephase und anschließend in der Abschmelzphase, wie das aus US-C-4 592 412 bekannt ist.

Der aus dem Flüssigabscheider kommende erste Strom weist gegenüber dem zweiten Strom eine höhere Konzentration an Nebenprodukten auf. Diese Nebenprodukte oder Verunreinigungen entstehen bei der katalytischen Oxidation von Orthoxylol oder Naphthalin zu PSA und verschlechtern die Qualität des Endprodukts. Zumindest ein Teil dieser Nebenprodukte läßt sich destillativ aus dem verflüssigten rohen PSA nur schwer abtrennen. Deshalb werden diese Nebenprodukte durch die thermische Vorbehandlung chemisch verändert und dabei teilweise polymerisiert. Die hierbei ablaufenden chemischen Reaktionen sind nicht genau bekannt. Um die gewünschten Reaktionen herbeizuführen, kann es sich empfehlen, eine reaktionsbeschleunigende Substanz zuzugeben, z. B. Na₂CO₃ oder KOH. Zu beachten ist jedoch, daß große Mengen an Reaktionsbeschleuniger ebenfalls wieder zu Nachteilen bei der Produktausbeute und auch zur Verschmutzung der destillativen Einrichtungen führen.

Der erste Verweilbehälter, welchem nur der erste PSA-reiche Strom zugeführt wird, kann ganz auf die Menge und die sonstigen Bedingungen dieses ersten Stroms ausgerichtet werden. Wenn man dem ersten Verweilbehälter Reaktionsbeschleuniger zugibt, genügt hierfür eine relativ geringe Menge von z. B. 10 bis 100 mg/kg, bezogen auf die Gesamtmenge des erzeugten PSA.

Es ist zweckmäßig, dem ersten Verweilbehälter mindestens einen zweiten Verweilbehälter nachzuschalten und diesem zweiten Behälter den aus dem ersten Behälter kommenden Strom zuzuführen. Der dem ersten Verweilbehälter zugeführte Reaktionsbeschleuniger wird dort üblicherweise nicht vollständig verbraucht und kann somit auch in den anschließenden Verweilbehältern wirksam werden. Es hat sich als zweckmäßig erwiesen, die Verweilzeit im ersten Behälter auf mindestens drei Stunden und in jedem weiteren Behälter auf mindestens zwei Stunden und vorzugsweise mindestens drei Stunden einzustellen. Aus den Festabscheidern kommt ein zweiter PSA-reicher Strom, der eine geringere Konzentration an Verunreinigungen enthält als der erste Strom, so daß der zweite Strom eine weniger intensive oder auch keinerlei Vorbehandlung benötigt. Es kann deshalb möglich sein, den zweiten Strom direkt in die destillative Reinigung zu geben oder den zweiten Strom in den zweiten Verweilbehälter oder direkt in einen dritten Verweilbehälter zu leiten.

Die Verunreinigungen reagieren bei der thermischen Behandlung, die mit oder ohne Zugabe von Reaktionsbeschleuniger erfolgt, unter Bildung von Makromolekülen, so daß sie in der anschließenden destillativen Reinigung leicht aus dem PSA-Produkt abtrennbar sind.

Ausgestaltungsmöglichkeiten des Verfahrens werden mit Hilfe der Zeichnung erläutert. Es zeigt:
Fig. 1 ein Fließschema des Verfahrens und
Fig. 2 eine Abwandlung des Fließschemas der Fig. 1.

Ein Gemisch aus Luft und Orthoxylol oder Naphthalin wird in der Leitung (1) herangeführt und tritt in einen Röhrenreaktor (2) ein, der in an sich bekannter Weise einen Katalysator, z. B. Vanadiumpentoxid, in von außen gekühlten Röhren enthält. In den Röhren liegen die Temperaturen im Bereich von 300 bis 500°C und vorzugsweise bei 350 bis 450°C. Als Kühlmittel dient z. B. eine Salzschmelze, was in der Zeichnung nicht im einzelnen dargestellt ist. Das im Reaktor (2) erzeugte Produktgemisch enthält Gase und Dämpfe, es wird in der Leitung (3) zunächst durch einen Flüssigabscheider (4) geführt. Das restliche Gemisch strömt durch die Leitung (5) zu einem von mehreren Festabscheidern (6), und ein PSA-armes Abgas zieht in der Leitung (7) ab.

Aus dem Flüssigabscheider (4) kommt in der Leitung (8) ein flüssiger, PSA-reicher Strom, der hier als "erster Strom" bezeichnet wird. Diesen ersten Strom gibt man dem ersten Verweilbehälter (11) auf, welcher Heizelemente (9) enthält und mit einer nicht dargestellten Rührvorrichtung ausgestattet ist. Zusätzlich kann man dem Behälter (11) durch die Leitung (10) Reaktionsbeschleuniger zugeben. Nach mehreren Stunden Verweilzeit im ersten Verweilbehälter (11) gelangt der teilweise thermisch vorbehandelte erste Strom durch die Leitung (15) zu einem zweiten Verweilbehälter (12), der im Prinzip genauso arbeitet wie der erste Behälter (11). Der aus den Festabscheidern (6) kommende zweite PSA-reiche Strom wird in der Leitung (16) abgezogen und kann ganz oder teilweise ebenfalls in den zweiten Verweilbehälter (12) gegeben werden. Möglich ist es aber auch, auf die thermische Vorbehandlung des zweiten Stroms ganz oder teilweise zu verzichten und die Bypassleitung (16a) zu benutzen.

Die aus dem zweiten Verweilbehälter (12) kommende Flüssigkeit wird in der Leitung (17) abgezogen und, gegebenenfalls gemischt mit der Flüssigkeit der Leitung (16a), einer ersten Destillationskolonne (18) zugeführt. Niedrigsiedende Verunreinigungen zieht man in der Leitung (19) ab und gibt das PSA-reiche Sumpfprodukt durch die Leitung (20) einer zweiten Destillationskolonne (21) auf. Schwersiedende Verunreinigungen zieht man durch die Leitung (22) aus dem Sumpf der Kolonne (21) ab und erhält weitgehend gereinigtes PSA-Produkt in der Leitung (23).

Die Beheizung der Verweilbehälter (11) und (12) sowie der Destillationskolonnen (18) und (21) kann in an sich bekannter Weise z. B. durch Wasserdampf oder Wärmeträgeröl erfolgen.

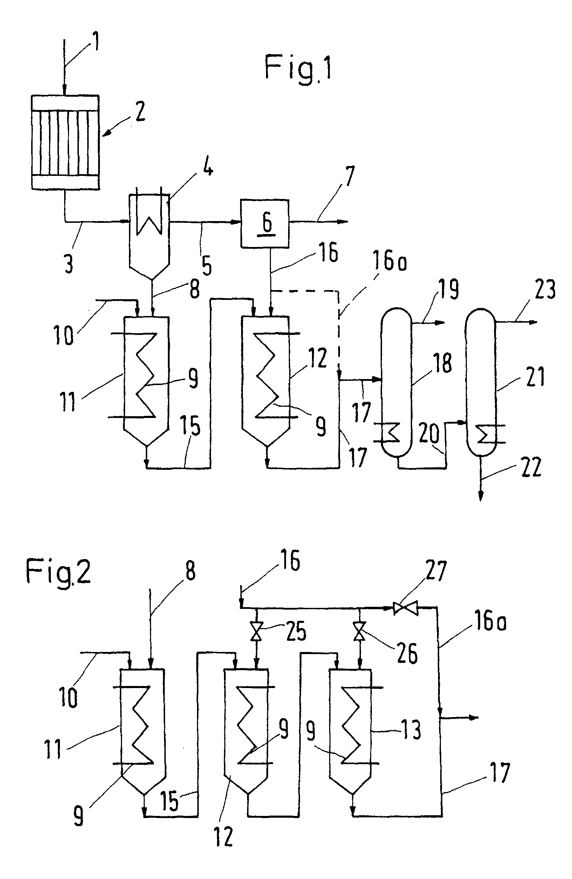

Bei der Verfahrensvariante der Fig. 2 wird der erste Strom, der in der Leitung (8) herangeführt wird, nacheinander durch die Verweilbehälter (11), (12) und (13) geführt. Der zweite Strom, der aus der Leitung (16) kommt, kann wahlweise ganz oder teilweise in die Verweilbehälter (12) und/oder (13) gegeben werden oder aber bei geschlossenen Ventilen (25) und (26) und geöffnetem Ventil (27) direkt der destillativen Reinigung zugeführt werden, die in Fig. 2 nicht dargestellt ist. Die übrigen Bezugsziffern haben die zusammen mit Fig. 1 gegebene Bedeutung. Für den ersten Strom liegt die gesamte Verweilzeit in den Behältern (11), (12) und (13) üblicherweise im Bereich von 6 bis 24 Stunden und zumeist 10 bis 18 Stunden.

### Beispiel:

In einer Anlage gemäß Fig. 1 werden im Röhrenreaktor (2) pro Stunde 3000 kg Roh-PSA erzeugt und dampfförmig in den Flüssigabscheider (4) geführt, in welchem eine Teilmenge flüssig abgeschieden wird; restliches Roh-PSA wird mittels 4 Festabscheidern (6) gewonnen. Dem 1 Verweilbehälter (11) führt man durch die Leitung (8) 1000 kg/h Roh-PSA zu und dem zweiten Behälter (12) durch die Leitung (16) 2000 kg/h; die Leitung (16a) entfällt. Jeder Behälter hat ein Fassungsvermögen von 24000 kg Roh-PSA. Als Reaktionsbeschleuniger werden 0,15 kg/h KOH in einer wäßrigen Lösung mit 10 Gew.-% KOH in den ersten Behälter gegeben. Die mittlere Verweilzeit beträgt im ersten Behälter 24 Stunden und im zweiten Behälter 8 Stunden, die Temperatur wird in beiden Behältern auf 270°C gehalten.

Der Strom in der Leitung (8) enthält pro kg PSA an störenden Verunreinigungen, die destillativ nicht (oder kaum) zu entfernen sind, 10 g, in der Leitung (15) findet sich noch 1 g, in der Leitung (16) ist ebenfalls 1 g/kg an diesen störenden Verunreinigungen zu finden und in der Leitung (17) noch 0,5 g/kg. Die beiden Destillationskolonnen (18) und (21) arbeiten bei einer Sumpftemperatur von 220°C. Durch die Leitung (19) zieht man pro Stunde 15 kg Leichtsieder (insbesondere Benzoesäure) und durch die Leitung (22) ebenfalls 15 kg/h schwersiedende Verunreinigungen einschließlich des eingesetzten KOH ab. Man erzeugt 2970 kg/h PSA, das in der Leitung (23) abgeführt wird.

## Patentansprüche

1. Verfahren zum Behandeln von rohem Phthalsäure-Anhydrid (PSA), welches katalytisch aus sauerstoffhaltigem Gas und Orthoxylol oder Naphthalin bei Temperaturen im Bereich von 300 bis 500°C in einem Reaktor erzeugt und in einem Gase und Dämpfe enthaltenden Gemisch aus dem Reaktor abgezogen wird, wobei man das Gemisch kühlt und flüssiges PSA durch eine thermische Vorbehandlung und eine destillative Reinigung leitet, **dadurch gekennzeichnet, daß** man
a) das Gase und Dämpfe enthaltende, aus dem Reaktor abgezogene PSA-haltige Gemisch zunächst durch einen gekühlten, kontinuierlich arbeitenden Flüssigabscheider leitet und einen ersten flüssigen, PSA-reichen Strom und, getrennt davon, ein restliches Gemisch aus Gasen und Dämpfen abzieht,
b) das restliche, PSA-haltige Gemisch durch mindestens einen von mehreren gekühlten Festabscheidern führt, wobei PSA in fester Form abgeschieden und, wieder verflüssigt, als zweiter PSA-reicher Strom aus den Festabscheidern abgezogen wird und wobei das Verhältnis der Mengen pro Zeiteinheit des ersten und zweiten Stroms 1 : 5 bis 2 : 1 beträgt,
c) den ersten PSA-reichen Strom bei Temperaturen von 250 bis 300°C zur thermischen Vorbehandlung unter Rühren durch einen ersten Verweilbehälter leitet, wobei die Verweilzeit im ersten Behälter mindestens drei Stunden beträgt, und
d) den zweiten PSA-reichen Strom unter Umgehung des ersten Verweilbehälters zusammen mit dem thermisch vorbehandelten ersten Strom zur destillativen Reinigung führt.

2. Verfahren nach Anspruch 1, **dadurch gekennzeichnet, daß** man den aus dem ersten Verweilbehälter abgezogenen ersten PSA-reichen Strom durch mindestens einen zweiten Verweilbehälter leitet, in welchem der Strom bei Temperaturen von 250 bis 300°C mindestens zwei Stunden lang gerührt wird.

3. Verfahren nach Anspruch 2, **dadurch gekennzeichnet, daß** man den zweiten PSA-reichen Strom ganz oder teilweise in den zweiten oder einen nachfolgenden Verweilbehälter leitet.

4. Verfahren nach Anspruch 1 oder einem der folgenden, **dadurch gekennzeichnet, daß** man dem ersten Verweilbehälter einen Reaktionsbeschleuniger zuführt.

## Claims

1. A method of treating crude phthalic acid anhydride (PSA) which is catalytically produced from oxygen-containing gas and orthoxylene or naphthalene at temperatures in the range from 300 to 500°C in a reactor and is withdrawn from the reactor in a mixture containing gases and vapours, wherein the mixture is cooled and liquid PSA is passed through stages of thermal pre-treatment and cleaning by distillation, **characterised in that**
a) the PSA-containing mixture containing gases and vapours which has been withdrawn from the reactor is first passed through a cooled, continuously operating liquid separator, and a first liquid stream rich in PSA and, separate therefrom, a residual mixture of gases and vapours are withdrawn,
b) the residual mixture containing PSA is passed through at least one of several cooled solid separators, wherein PSA is separated in solid form and, having been liquefied again, is withdrawn from the solid separators as a second stream rich in PSA, and wherein the ratio of the amounts per unit of time of the first and the second stream is 1 : 5 to 2 : 1,
c) the first stream rich in PSA is passed at temperatures of 250 to 300°C with stirring through a first delay tank for thermal pre-treatment, the dwell time in the first tank being at least three hours, and
d) bypassing the first delay tank, the second stream rich in PSA is supplied to the cleaning by distillation together with the thermally pre-treated first stream.

2. A method according to Claim 1, **characterised in that** the first stream rich in PSA which has been withdrawn from the first delay tank is passed through at least one second delay tank, in which the stream is stirred for at least two hours at temperatures of 250 to 300°C.

3. A method according to Claim 2, **characterised in that** the second stream rich in PSA is introduced wholly or in part into the second or a subsequent delay tank.

4. A method according to Claim 1 or any one of the preceding claims, **characterised in that** a reaction promoter is supplied to the first delay tank.

## Revendications

1. Procédé de traitement d'anhydride phtalique (AP) brut qui est produit catalytiquement à partir de gaz contenant de l'oxygène et d'orthoxylène ou de naphtalène à des températures de l'ordre de 300 à 500°C dans un réacteur et qui est soutiré du réacteur dans un mélange contenant des gaz et des vapeurs, le mélange étant refroidi et du AP liquide étant envoyé dans un prétraitement thermique et dans une purification par distillation, **caractérisé en ce que**
a) on envoie le mélange contenant des gaz et des vapeurs, soutiré du réacteur et contenant de l'AS d'abord dans un séparateur de liquides refroidi et fonctionnant en continu et on soutire un premier courant liquide riche en AS et, indépendamment de cela, un mélange restant constitué de gaz et de vapeurs,
b) on envoie le mélange restant contenant de l'AS dans au moins l'un de plusieurs séparateurs de matières solides refroidis, de l'AS étant séparé sous forme solide et reliquéfié, étant soutiré en tant que deuxième courant riche en AS des séparateurs de matières solides, et le rapport des quantités par unité de temps du premier et du deuxième courant va de 1:5 à 2:1,
c) on envoie le premier courant riche en AS à des températures de 250 à 300°C au prétraitement thermique sous agitation dans une première cuve de séjour, la durée de séjour dans la première cuve étant d'au moins trois heures, et
d) on envoie le deuxième courant riche en AS en contournant la première cuve de séjour ensemble avec le premier courant prétraité thermiquement à la purification par distillation.

2. Procédé suivant la revendication 1, **caractérisé en ce que** l'on envoie le premier courant riche en AS soutiré de la première cuve de séjour dans au moins une deuxième cuve de séjour, dans laquelle le courant est brassé pendant au moins deux heures à des températures de 250 à 300°C.

3. Procédé suivant la revendication 2, **caractérisé en ce que** l'on envoie le deuxième courant riche en AS en tout ou en partie dans la deuxième cuve de séjour ou dans une cuve de séjour venant ensuite.

4. Procédé suivant la revendication 1 ou l'une des suivantes, **caractérisé en ce que** l'on apporte à la première cuve de séjour un accélérateur de réaction.
